# EUROPÄISCHE PATENTSCHRIFT

(11) **EP 1 208 410 B1**
(45) Veröffentlichungstag und Bekanntmachung des Hinweises auf die Patenterteilung: **22.12.2004**
(21) Anmeldenummer: 00934997.8
(22) Anmeldetag: 10.05.2000
(51) Int. Cl.: G05B 19/4099, A61F 2/28, A61F 2/30

(54) **VERFAHREN ZUR GENERIERUNG PATIENTENSPEZIFISCHER IMPLANTATE**
METHOD FOR GENERATING PATIENT-SPECIFIC IMPLANTS
PROCEDE POUR LA GENERATION D'IMPLANTS SPECIFIQUES AUX PATIENTS

(30) Priorität: 11.05.1999 DE 19922279
(43) Veröffentlichungstag der Anmeldung: 29.05.2002
(73) Patentinhaber: 3di GmbH, 07745 Jena (DE); Körbs, Thomas, 07629 Hermsdorf (DE); Nagel, Sebastian, 07743 Jena (DE); Schied, Ralf, 06618 Naumburg (DE); Litschko, Peter, 06618 Naumburg (DE)
(72) Erfinder: LITSCHKO, Peter, D-06618 Naumburg (DE); HENNING, Torsten, D-07743 Jena (DE); BEINEMANN, Jörg, D-99425 Weimar (DE); FRIED, Wolfgang, D-07749 Jena (DE); LINSS, Werner, D-07743 Jena (DE)
(74) Vertreter: Pfeiffer, Rolf-Gerd
(86) Internationale Anmeldenummer: PCT/EP2000/004166
(87) Internationale Veröffentlichungsnummer: WO 2000/068749

(56) Entgegenhaltungen:
- US-A- 5 360 446
- US-A- 5 370 692
- US-A- 5 448 489
- US-A- 5 554 190
- US-A- 5 741 215
- US-A- 5 769 092
- US-A- 5 798 924
- US-A- 5 824 085

## Beschreibung

Die Erfindung betrifft die Generierung patientenspezifischer Implantate ausgehend von vorliegenden Untersuchungsergebnissen vom Patienten aus bildgebenden Verfahren der Medizintechnik.
Es ist seit langem möglich, körperfremde Materialien (Implantate) einzusetzen, um Defekte an Organismen zu schließen. Aktueller Stand der Technik ist die Erzeugung von patientenspezifisch angepaßten Hartgewebe-Implantaten entweder durch Generierung der Implantate während der Operation, unter Verwendung von real vorhandenen Zwischenmodellen oder seit kurzem unter Verwendung von CAD/CAM-Technologien als Hilfsmittel zur computergestützten Konstruktion. Grundlage der Generierung bilden dabei zunehmend bildgebende Verfahren der Medizintechnik, z. B. Computertomographie, Kernspintomographie, Sonographie usw.
Es ist allgemeine medizinische Praxis (z. B. US 4.097.935, US 4976.737), begrenzt form- bzw. bearbeitbare Metallnetze, Metallplatten, leicht formbare Materialien, welche nach kurzer Zeit aushärten (beispielsweise Kunstharz) und körpereigene Materialien des Patienten zur Implantation zu verwenden, mit denen der Defekt während der Operation geschlossen wird, d. h. das Implantat wird während der Operation erstellt, bearbeitet und an den Defekt angepaßt. Metallische Implantate, wie Netze, Platten etc., können allerdings bei einer späteren Diagnostik am Patienten sehr störend sein und sogar einzelne Untersuchungsmethoden, besonders bei großen Defektarealen, für die Zukunft ausschließen. Der Operationsverlauf wird gewöhnlich durch die Behandlungssituation selbst und durch die Erfahrung des Arztes bestimmt; die Möglichkeit einer vorherigen konkreten Operationsplanung zum Einsatz des Implantats ist in diesen Fällen so gut wie nicht gegeben. Deshalb werden für den Operierten nicht selten Nachbehandlungen erforderlich, die eine zusätzliche physische und psychische Belastung des Patienten darstellen. Darüber hinaus sind einige Materialien, wie Kunststoffe, die leicht formbar sind und/oder mit relativ geringem Aufwand hergestellt werden, bezüglich ihrer Belastbarkeit und Dauerbeständigkeit in ihren Einsatzmöglichkeiten beschränkt. Hinzu kommt der Wunsch des Patienten nach einem ästhetischen Erscheinungsbild, das in vielen Fällen schwer realisierbar ist.
Es ist auch möglich ("Stereolithographic biomodelling in craniomaxillofacial surgery, a prospective trial", Journal of Cranio-Maxillofacial Surgery, 27, 1999 oder US 5.370.692 oder US 5.452.407 oder US 5.741.215), den Implantatentwurf mit der Generierung eines physischen 3D-Zwischenmodells, z. B. durch stereolithographische Methoden auf der Basis der eingangs genannten bildgebenden medizinischen Untersuchungsverfahren, zu beginnen. Anschließend wird das Implantat manuell in den Defekt unter Einsatz plastisch formbarer Materialien modelliert und erst danach in der endgültigen Form aus dem Implantatmaterial erstellt. Bevorzugt wird dabei eine Fertigung des Implantats in Materialien mit höherer Festigkeit, wie Titan.
Darüber hinaus ist es bekannt ("Schädelimplantate - computergestützte Konstruktion und Fertigung", Spektrum der Wissenschaft, Februar 1999; "Die Rekonstruktion kraniofazialer Knochendefekte mit individuellen Titanimplantaten", Deutsches Ärzteblatt, September 1997), aus den Daten bildgebender Untersuchungsverfahren des Patienten ein einfaches 3D-CAD Patientenmodell zu generieren und aus diesem unter Verwendung einfacher konstruktiver Methoden das Implantat manuell am Rechner zu entwerfen. Anschließend wird das Implantat für den operativen Einsatz durch CNC-Verfahren hergestellt.
Alle vorgenannten Verfahren sind jedoch mit den Nachteilen behaftet, daß das Ergebnis der Implantat-Modellierung überwiegend von den Erfahrungen, von den Fähigkeiten und vom "künstlerischen" Geschick der Person, welche dieses Implantat generiert bzw. erstellt, bestimmt wird. Die Fertigung, ausgehend von den vorliegenden Daten bis hin zum operativ einsetzbaren und paßfähigen Implantat ist mit einem hohen Kosten- und Zeitaufwand verbunden, der sich im Fall der Anfertigung eines sog. Zwischenmodells sogar noch weiter vergrößert. Die Anfertigung eines Implantats während einer Operation erfordert einen entsprechend hohen zeitlichen und organisatorischen Aufwand für den operativen Eingriff und stellt damit nicht zuletzt für den Patienten eine sehr hohe physische und psychische Belastung dar. Außerdem ist es schwieriger, ein nicht zur Defektstelle der Patienten paßfähiges Implantat formschlüssig unter medizinischen und ästhetischen Gesichtspunkten operativ einzusetzen. Oftmals entscheiden auch hier die besonderen Fähigkeiten und Erfahrungen des Chirurgen über das Ergebnis der Operation. Die erstgenannten Verfahren sind in der Praxis und im Rahmen der Kliniksroutine nur bei kleineren und/oder gering strukturierten Defekten anwendbar und gelangen zur Formung und Anpassung bei komplizierten Defekten und Implantaten schnell an ihre herstellungstechnischen Grenzen.
Der operative Aufwand wird in hohem Maße durch die Paßfähigkeit des Implantats an die Defektstelle bestimmt. Gerade bei einer Implantatherstellung über Zwischenmodelle kann aber diese Genauigkeit durch Abformung vom Zwischenmodell zusätzlich beeinträchtigt werden.
In komplizierten Fällen müssen die Implantate in langwierigen und extrem zeitaufwendigen Prozessen, ggf. über mehrere Zwischenstufen, hergestellt werden. In diesem relativ großen Zeitraum verändert sich unter Umständen zwischenzeitlich das Defektgebiet beim Patienten. Diese Veränderungen können in der Praxis für eine möglichst genaue Paßfähigkeit des Implantats nicht ausreichend berücksichtigt werden und vergrößern den Aufwand für den operativen Einsatz weiter.
Aus der Sicht des Arztes sowie auch des Patienten wäre es wünschenswert, die Implantate in kürzest möglicher Zeit, auch in Hinsicht auf den operativen Eingriff, und mit hoher Paßfähigkeit zur Defektstelle am Patienten herzustellen. Dem behandelnden Chirurgen sollten für eine vorherige Operationsplanung vor dem Patienteneingriff konkrete Angaben nicht nur zur Defektstelle am Patienten, sondern gleichermaßen zur Größe und Form des einzusetzenden Implantats, vorliegen.

Der Erfindung liegt die Aufgabe zugrunde, unabhängig von der Größe, Form und Kompliziertheit der Defektstelle des Patienten ein funktionell und ästhetisch exakter an diese angepaßtes Implantat zu generieren, das in kürzerer Zeit und mit geringerem Aufwand sowohl hergestellt als auch beim Patienten in einem Operationsvorgang eingesetzt werden kann. Das Verfahren sollte mit gleicher Genauigkeit für alle Formen, Größen sowie für alle in Frage kommenden Implantatmaterialien anwendbar sein.

Erfindungsgemäß wird das virtuelle dreidimensionale Modell des Patienten, welches bekannterweise aus vorliegenden (zweidimensional) aufgenommenen Bilddaten zumindest für dessen Implantat- und Umgebungsbereiches gebildet wurde, mit realmedizinischen Referenzdaten verglichen. Aus diesem Vergleich werden das für den Patienten geeignetste Modell bzw. ein dem Modell des Patienten ähnlichstes Referenzmodellobjekt ausgewählt sowie nach diesem ein virtuelles Implantatmodell generiert. Aus den im Rechner vorliegenden virtuellen Implantatmodelldaten werden unmittelbar CNC-Steuerdaten für die programmgesteuerte Herstellung des Implantats erzeugt. Der Vergleich mit realmedizinischen Referenzdaten kann sowohl in einer Datenbank mit dort gespeicherten medizinischen Daten einer Anzahl von Probanden (anderer Personen) als auch unter Verwendung von Daten vom Patienten selbst erfolgen, dessen Körpersymmetrie (insbesondere doppelt vorhandene spiegelsymmetrische Körperbereiche) zur Auswahl bzw. Generierung eines Patientenreferenzmodells berücksichtigt werden. Auch Daten aus früheren Untersuchungen des Patienten, welche den Defekt noch nicht aufweisen oder die auf dessen Veränderung hinweisen, können für diesen Vergleich herangezogen werden.
Auf diese Weise wird das Implantat virtuell unter ästhetischen und funktionellen Aspekten und lediglich mit rechentechnischem Aufwand (Software) in sehr kurzer Zeit patientenspezifisch modelliert sowie für beliebige Form, Größe und Kompliziertheitsgrad des notwendigen Implantats sehr exakt an die Form der Defektstelle des Patienten angepaßt. Anhand des mit CAD/CAM-Unterstützung generierten und an die Defektstelle bzw. an repräsentative Vergleichsdaten zu diesem angepaßten virtuellen Implantatmodells kann der behandelnde Chirurg sehr konkrete Angaben für eine objektive Operationsplanung am virtuellen Modell erhalten und bereits im Vorfeld des Eingriffs den Operationsverlauf simulieren, wodurch die eigentliche Operation und deren Verlauf besser vorbereitet, durchgeführt und im Erfolg realistischer eingeschätzt und ggf. auch a priori mit dem Patienten beraten und abgestimmt werden kann. Aus dem virtuellen Referenzmodell des Patienten wird durch Anwendung mathematischer Algorithmen das Implantatmodell extrahiert und daraus unmittelbar die Steuerdaten für das die Defektstelle ausfüllende bzw. verschließende Implantat abgeleitet, so daß das Implantat mit den Vorteilen der an sich bekannten CNC-Steuerung sofort programmgesteuert und ohne erforderliche Zwischen- oder Probemodelle (insbesondere zur Abformung, zu Testzwecken, zur Nachbesserung, zur Korrektur sowie ggf. zur Neuanfertigung) körperlich hergestellt werden kann. Mit CNCgesteuerten Fertigungsmaschinen, denen die Daten rechnergestützt übergeben werden, sind Implantate von nahezu beliebiger Form und Größe, wie auch aus beliebigen Materialien, einschließlich Keramiken und Titan, herstellbar, so daß bei jedem Patienten das Implantat hinsichtlich der erforderlichen Eigenschaften (Funktion, Belastbarkeit, Resorbierbarkeit, Dauerbeständigkeit, ästhetisches Aussehen, biologische Verträglichkeit etc.) gewählt werden kann. Die so in kürzester Zeit gewährleistete Implantatherstellung, die auch ebenso schnell unter neuen oder geänderten Gesichtspunkten des operativen Eingriffs wiederholt werden kann, verringert den zeitlichen und organisatorischen Ablauf im klinischen Betrieb sowie den Aufwand für den Chirurgen und das gesundheitliche Risiko für den Patienten. Besonders aus Sicht des Patienten ist ferner vorteilhaft, daß durch die exakte Anpassung des herzustellenden Implantats an den Defektbereich eine hohe Ästhetik des implantierten Defektareals erreicht und chirurgische Korrekturen, Nachbesserungen sowie sonstige Folgeoperationen vermieden oder zumindest in Umfang und Zahl verringert werden.

Die Erfindung soll nachstehend anhand von in der Zeichnung dargestellten Ausführungsbeispielen näher erläutert werden. Es zeigen:
Fig. 1 eine allgemeine Verfahrensübersicht,
Fig. 2 eine detaillierte Verfahrensübersicht,
Fig. 3 eine Aufbereitung medizinischer 2D-Bilddaten,
Fig. 4 eine Erzeugung des 3D-Patientenmodells,
Fig. 5 ein Invertierungsmodell,
Fig. 6 ein 3D-Referenzmodell,
Fig. 7 ein 3D-Implantatmodell

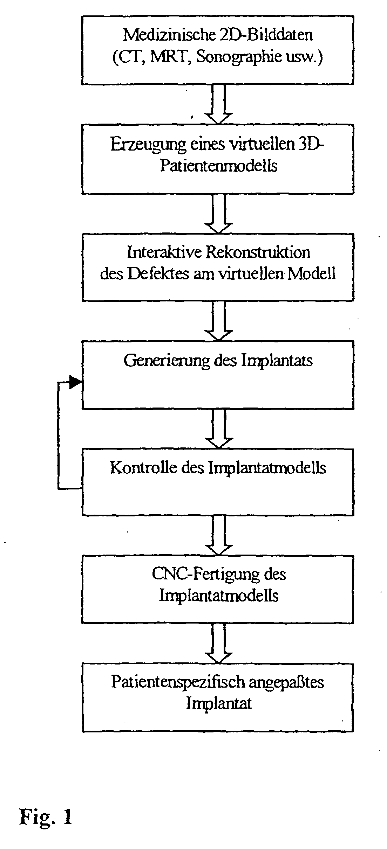

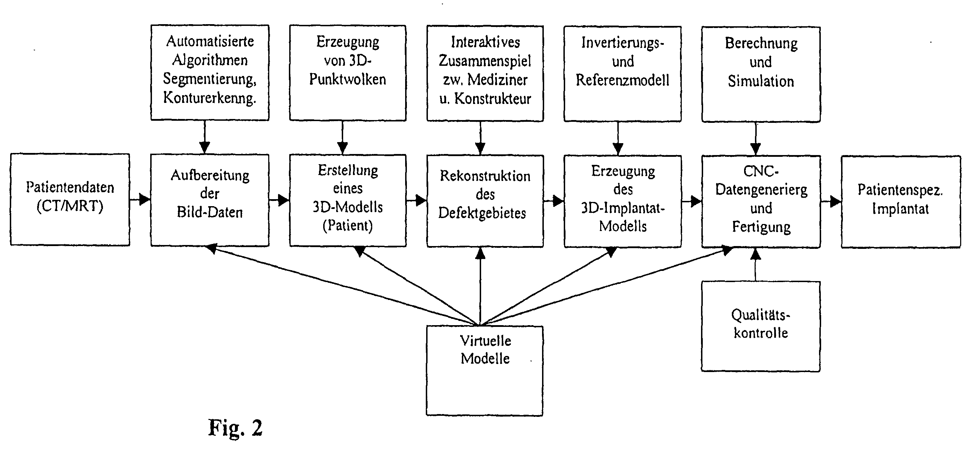

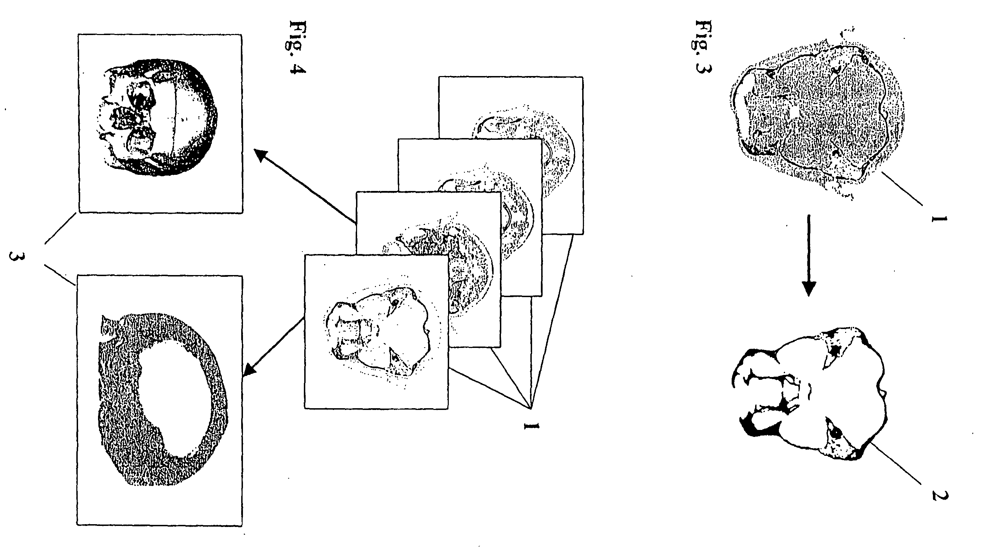

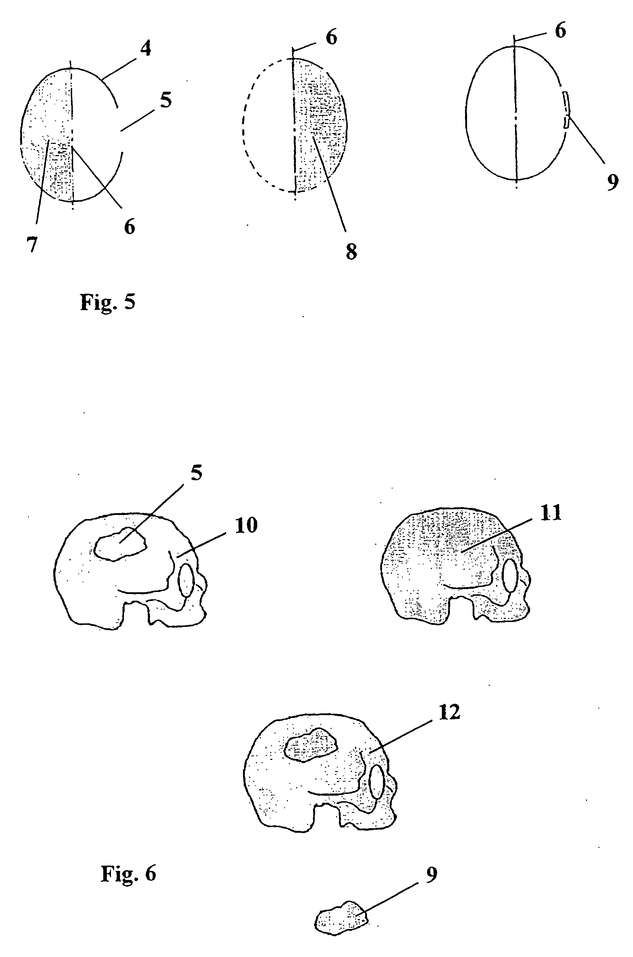

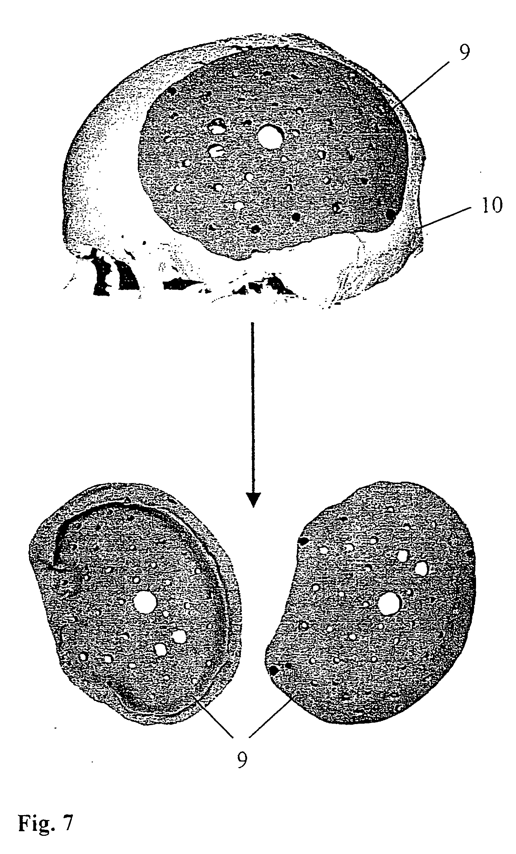

Als Beispiel soll der Fall eines Patienten mit einem großflächigen, komplizierten Defekt (z. B. infolge eines Unfalls, Tumors usw.) in der oberen Schädelhälfte angeführt werden. In den Figuren 1 und 2 sind zur Veranschaulichung des erfindungsgemäßen Verfahrens sowohl eine allgemeine als auch eine detaillierte blockdiagrammartige Übersicht dargestellt.
Zur genauen Diagnostik und späteren Implantatgenerierung werden in der Radiologischen Abteilung eines Krankenhauses (beispielsweise durch Computertomographie oder Kemspintomographie) vom Patienten medizinische 2D-Bilddaten 1 (2D-Schichtbilder) eines Defektareals 5 und von dessen Umgebung aufgenommen (vgl. Fig. 3).
Unter Verwendung von mathematischen Bildverarbeitungs-Algorithmen erfolgt in den 2D-Bilddaten 1 zunächst eine Konturerkennung und anschließend eine Segmentierung mit dem Ziel, die Hartgewebsbereiche (Knochen) zu detektieren. Im Ergebnis der Konturerkennung und Segmentierung entstehen 2D-Bilddaten 2, aus denen durch entsprechende räumliche Anordnung ein virtuelles 3D-Patientenmodell 3 (Punktmodell) zumindest des Defektareals 5 mit Umgebung gebildet wird.
Im Zusammenspiel zwischen Mediziner und Konstrukteur wird an diesem virtuellen 3D-Patientenmodell 3 das Defektgebiet unter Verwendung speziell für diesen Zweck anwendbarer Computerprogramme genau definiert und markiert.
Dem Konstrukteur des Implantates stehen im nächsten Schritt verschiedene Methoden zur Generierung paßgenauer Implantate zur Verfügung. Diese sind:
1. Liegt an einem 3D-Patientenmodell 4 (in Fig. 5 als Querschnitt dargestellt) das Defektareal 5 komplett in einer Körperhälfte, d. h. komplett auf einer Kopfseite, so lassen sich die Daten dieser Körperseite mit dem Defektareal 5 unter Ausnutzung der Symmetrieeigenschaften des menschlichen Körpers aus den Daten einer intakten Seite 7 des 3D-Patientenmodells 4 durch eine Invertierung 8 nachbilden (Spiegelung der intakten Seite 7 an der Symmetrieebene 6). Nach der Invertierung erfolgt durch Anwendung von mathematischen Algorithmen, die hier nicht näher ausgeführt werden sollen, die Extraktion eines virtuellen Implantatmodells 9.
2. Liegt an einem 3D-Patientenmodell 10 (in Fig. 6 in Seitenansicht gezeigt) das Defektareal 5 auf der Symmetrieebene des menschlichen Körpers oder sind die Daten der intakten Seite aus irgendeinem Grund nicht verwendbar, so läßt sich das virtuelle Implantatmodell 9 über ein 3D-Referenzmodell 11 generieren. Dazu wird anhand eines Vergleiches von spezifischen Merkmalen des 3D-Patientenmodells 10 in einer Referenzdatenbank eine Auswahl an ähnlichen Modellen unter Berücksichtigung mathematischer, funktioneller, medizinischer und ästhetischer Gesichtspunkte getroffen. Aus dieser Auswahl wird letztlich, vorzugsweise unter besonderer medizinischer Begutachtung, das 3D-Referenzmodell 11 selektiert. Durch Überlagerung des 3D-Referenzmodells 11 und des 3D-Patientenmodells 10 entsteht ein virtuelles 3D-Patientenmodell 12, aus dem wiederum, wie in Punkt 1 beschrieben, das virtuelle Implantatmodell 9 am Rechner generiert wird.
3. In speziellen Fällen, beispielsweise wenn der Defekt teilweise auf der Symmetrieebene liegt, können beide Methoden (Invertierung nach Punkt 1 und Datenbankvergleich gemäß Punkt 2) nacheinander angewendet und die Ergebnisse zu einem 3D-Referenzmodell für die Implantatmodellierung kombiniert werden.
Die Auswahl und/oder Bildung des 3D-Referenzmodells nach einem oder mehreren der vorgenannten Methoden und die Generierung des virtuellen Implantatmodells aus dem 3D-Referenzmodell erfolgen rein rechentechnisch. Mit dieser Bearbeitung ist sowohl eine sehr schnelle als auch paßgenaue Generierung und anschließende Herstellung des Implantats für den operativen Einsatz am Patienten gegeben.
Das vorliegende virtuelle Implantatmodell 9 (Fig. 7) wird im Anschluß an die Generierung verschiedenen Prozeduren unterzogen. Dazu können z. B. Festigkeitsberechnungen, Simulationen für die medizinische Operationsplanung und Fertigung, sowie das Anbringen von Markierungen (Bohrungen, Befestigungen o. ä.), Qualitätskontrollen usw. gehören.
Nach dem Entwurf des virtuellen Implantatmodells 9 erfolgt eine Erzeugung/ Simulation von CNC-Steuerdaten für die körperliche Implantatfertigung und die Überführung des virtuellen Implantatmodells in ein einsatzfähiges Implantat.

### Bezugszeichenliste

- 1 -: Medizinische 2D-Bilddaten
- 2 -: 2D-Bilddaten nach Konturerkennung und Segmentierung
- 3 -: 3D-Patientenmodell (Punktmodell)
- 4 -: 3D-Patientenmodell (Querschnitt)
- 5 -: Defektareal
- 6 -: Symmetrieebene des menschlichen Körpers
- 7 -: Intakte Seite des 3D-Patientenmodells
- 8 -: Invertierung der intakten Seite 7
- 9 -: Implantatmodell
- 10 -: 3D-Patientenmodell (Seitenansicht)
- 11 -: 3D-Referenzmodell
- 12 -: 3D-Patientenmodell

## Patentansprüche

1. Verfahren zur Generierung patientenspezifischer Implantate, bei dem aus vom Patienten vorliegenden Bilddaten zumindest des Implantat- und Umgebungsbereiches ein virtuelles dreidimensionales Modell generiert und das Implantat anhand von CNC-Steuerdaten für den operativen Einsatz beim Patienten hergestellt wird, wobei das virtuelle dreidimensionale Modell des Patienten mit realmedizinischen Referenzdaten verglichen und aus diesen das für den Patienten geeignetste bzw. ein dem Modell des Patienten ähnlichstes Referenzmodellobjekt ausgewählt oder gebildet wird, wobei anhand eines Vergleiches von spezifischen Merkmalen des 3D-Patientenmodells (10) in einer Referenzdatenbank eine Auswahl an ähnlichen Modellen unter Berücksichtigung mathematischer, funktioneller, medizinischer und ästhetischer Gesichtspunkte getroffen wird und aus dieser Auswahl unter besonderer medizinischer Begutachtung, das 3D-Referenzmodell (11) selektiert wird und durch Überlagerung des 3D-Referenzmodells (11) und des 3D-Patientenmodells (10) ein virtuelles 3D-Patientenmodell (12) generiert wird, wobei nach diesem 3D-Patientenmodell (12) ein virtuelles Implantatmodell generiert wird und wobei die virtuellen Daten des Implantatmodells als Steuerdaten für die programmgesteuerte Herstellung des Implantats verwendet werden.

2. Verfahren nach Anspruch 1, **dadurch gekennzeichnet, daß** das virtuelle dreidimensionale Modell des Patienten mit realmedizinischen Referenzdaten einer Datenbank verglichen und aus den in dieser gespeicherten Daten das für den Patienten geeignetste bzw. ein dem Modell des Patienten ähnlichstes Referenzmodellobjekt ausgewählt wird.

3. Verfahren nach Anspruch 2, **dadurch gekennzeichnet, daß** von den in der Datenbank gespeicherten Daten zunächst mehrere dem Modell des Patienten ähnliche Referenzmodellobjekte selektiert und aus diesen unter Berücksichtigung weiterer Auswahlkriterien, wie medizinische Gutachten, das für den Patienten geeignetste bzw. ein dem Modell des Patienten ähnlichstes Referenzmodellobjekt ausgewählt wird.

4. Verfahren nach Anspruch 1, **dadurch gekennzeichnet, daß** das virtuelle dreidimensionale Modell des Patienten mit Daten des Patienten selbst, vorzugsweise unter Berücksichtigung der Körpersymmetrie, insbesondere doppelt vorhandener spiegelsymmetrischer Körperbereiche und/oder unter Berücksichtigung älterer Daten des Patienten, verglichen wird, und daß aus diesen Daten das für den Patienten geeignetste bzw. ein dem Modell des Patienten ähnlichstes Referenzmodellobjekt ausgewählt oder gebildet wird.

5. Verfahren nach Ansprüchen 1, 2 und 4, **dadurch gekennzeichnet, daß** das virtuelle dreidimensionale Modell des Patienten sowohl mit den realmedizinischen Referenzdaten einer Datenbank als auch mit den Daten vom Patienten selbst verglichen wird, und daß aus allen diesen Daten das für den Patienten geeignetste bzw. ein dem Modell des Patienten ähnlichstes Referenzmodellobjekt ausgewählt oder gebildet wird.

## Claims

1. Method for generating patent-specific implants, in which a virtual three-dimensional model is generated from the image data taken from the patient and from at least the implant area and its environment and the implant is manufactured by virtue of CNC-data for the operative use on the patient, wherein the virtual three-dimensional model of the patient is compared to real medical reference data, and therefrom a reference model object best suited for the patient, respectively, a model most resembling the patient is selected or formed, wherein by way of comparison of specific features of the three-dimensional patient model (10) in a reference database a selection of similar models is carried out under consideration of mathematical, functional, medical, and aesthetic aspects, and from this selection the three-dimensional reference model (11) is selected under specific medical expert opinion, and a virtual three-dimensional patient model (12) is generated by superimposition of the three-dimensional reference model (11) with the three-dimensional patient model (10), and wherein a virtual implant model is generated according to the three-dimensional patient model (12) and that the virtual data of the implant model are used as control data for a program controlled manufacture of the implant.

2. Method as claimed in claim 1, **characterized in that** the virtual three-dimensional model of the patient is compared to real medical reference data of a database and from these data stored in the database a reference model best suited for the patient and best resembling the model of the patient, respectively, is selected.

3. Method as claimed in claim 2, **characterized in that** at first a plurality of reference models objects resembling the model of the patient are selected from the data stored in the database and from the selected ones a reference model object best suited for the patient and mostly resembling the model of the patient, respectively, is selected under consideration of further selection criteria such as medical expert opinion.

4. Method as claimed in claim 1, **characterized in that** the virtual three-dimensional model of the patient is compared with data from the patient him/herself preferably under consideration of body symmetry, in particular of mirror-symmetrical body ranges being doubly present and/or under consideration of older data from the patient and that from these data a reference model object best suited for the patient and most resembling the model of the patient, respectively, is selected or formed.

5. Method as claimed in claims 1, 2, and 4, **characterized in that** the virtual three-dimensional model of the patient is compared both, with the real medical reference data of a database and with the data from the patient him/herself, and **in that** from all these data a reference model object best suited for the patient and most resembling the model of the patient, respectively, is selected or formed.

## Revendications

1. Procédé pour la génération d'implants personnalisés spécifiques aux patients permettant de créer, à partir des données d'image relevées pour au moins la région à restaurer par l'implant et les zones voisines, un modèle tridimensionnel virtuel et de façonner à l'aide de données de commande CNC l'implant solide à poser par l'intervention chirurgicalé, le modèle tridimensionnel virtuel du patient étant comparé aux données médicales réelles de référence pour sélectionner ou générer le modèle de référence le mieux approprié et le plus proche du modèle personnalisé spécifique du patient, après avoir sélectionné des modèles similaires par comparaison des caractéristiques spécifiques du modèle personnalisé 3D (10), mémorisées dans la base de données de référence, avec prise en considération des aspects fonctionnels, médicaux et esthétiques, puis sélectionné, sur la base d'expertises médicales spécifiques, le modèle de référence 3D (11) qui sera superposé au modèle personnalisé 3D (10) pour finalement pourvoir créer un modèle virtuel personnalisé 3D (12) à partir duquel il sera possible de générer un modèle d'implant virtuel dont les données virtuelles peuvent être utilisées comme données de commande pour façonner par commande programmée l'implant solide à poser chez le patient.

2. Procédé suivant la revendication 1, **caractérisé en ce que** le modèle tridimensionnel virtuel du patient est comparé aux données médicales réelles de référence mémorisées dans une base de données, et que l'objet du modèle de référence le mieux approprié au patient ou le plus proche du modèle personnalisé du patient sera sélectionné à partir de ces données mémorisées dans la base.

3. Procédé suivant la revendication 2, **caractérisé en ce que** plusieurs objets de modèles de référence similaires au modèle personnalisé du patient sont sélectionnés d'abord parmi les données mémorisées dans la base de donnée, et que se trouve sélectionné, en prenant en considération d'autres critères tels que des expertises médicales, le modèle le mieux approprié au patient ou le modèle de référence le plus proche au modèle personnalisé du patient.

4. Procédé suivant la revendication 1, **caractérisé en ce que** le modèle tridimensionnel personnalisé virtuel du patient est comparé avec les données du patient, de préférence en prenant en considération la symétrie corporelle, en particulier des zones symétriques existant en double et/ou en tenant compte de données plus anciennes du patient , et qu'à partir de ces données sera sélectionné et créé le modèle le mieux approprié au patient ou le modèle de référence le plus proche au modèle personnalisé du patient.

5. Procédé suivant les revendications 1, 2 et 4, **caractérisé en ce que** le modèle tridimensionnel virtuel du patient se trouve comparé aussi bien avec les données de référence médicales réelles mémorisées dans une base de données qu'avec les données personnalisées du patient, et qu'à partir de toutes ces données sera sélectionné le modèle le mieux approprié au patient ou l'objet de modèle de référence le plus proche au modèle personnalisé du patient.
